# EUROPEAN PATENT SPECIFICATION

(11) **EP 0 796 431 B1**
(45) Date of publication and mention of the grant of the patent: **29.01.2003**
(21) Application number: 95914368.6
(22) Date of filing: 30.03.1995
(51) Int. Cl.: G01N 33/543, G01N 27/26

(54) **IMMUNOASSAY METHOD AND APPARATUS WITH SOLID PHASE CARRIER SITUATED BETWEEN TWO ELECTRODES**
IMMUNOTESTVERFAHREN UND GERÄT MIT EINEM, ZWISCHEN ZWEI ELEKTRODEN PLAZIERTEM FESTPHASENTRÄGER
PROCEDE DE DOSAGE IMMONOLOGIQUE ET APPAREIL ASSOCIE COMPRENANT UNSUPPORT EN PHASE SOLIDE PLACE ENTRE DEUX ELECTRODES

(30) Priority: 31.03.1994 FI 941526
(43) Date of publication of application: 24.09.1997
(73) Proprietor: HAKALEHTO, Elias, 70110 Kuopio (FI)
(72) Inventor: HAKALEHTO, Elias, 70110 Kuopio (FI)
(74) Representative: Pitkänen, Hannu Alpo Antero
(86) International application number: FI9500172
(87) International publication number: WO95027207

(56) References cited:
- WO-A-87/03095
- DE-C- 2 801 026
- US-A- 4 081 334
- US-A- 5 137 827
- SCIENCE, Vol. 240, No. 4856, (27-05-1988), DEAN G. HAFEMAN et al., "Light-Addressable Potentiometric Sensor for Biochemical Systems", page 1182-1185.

## Description

### Object of the invention

The invention relates to an apparatus for a carrier based immunoassay and to a method for using the apparatus.

### Background art

There are numerous immunoassays based on a solid carrier that are generally known to a person skilled in the art. In rapid tests used in immunoassay a carrier is often an even surface attached to a test strip. A reactant, such as an antigen or antibody, is bound to the solid carrier, and the first reactant bound to the carrier is then contacted with a second reactant, for example a corresponding antibody or antigen, reacting with it, whereafter the bonding of the second reactant with the carrier via the first reactant is assayed either directly or indirectly. A tracer connected to a reactant, for example an antibody, is often utilized in the detection. This tracer may be for example a fluorescent agent, a radioactive substance or an enzyme, which may then be detected by means of fluorescence, by measuring the radioactivity or by indicating the enzymatic reaction.

The problem with these generally known immunoassay methods is often that the concentration of the agents in the reaction is so low that it is difficult to detect them, and therefore it would be desirable to enrich the tracer used in the detection. Another problem in immunoassays is for example the formation of aggregates, i.e. nonspecific binding. In order to reduce nonspecific binding and to isolate unreacted agents, it is usually necessary to perform thorough washes, whereupon the entire surface of the carrier should be treated in the same manner.

### Summary of the invention

The purpose of the present invention is to eliminate the aforementioned problems. In order to achieve this, the invention provides an apparatus as defined in the appended claims which comprises a positive and a negative electrode placed in a container for electrically conductive liquid and connected to a power supply, and a solid carrier placed between them. The apparatus is applicable in immunoassay methods which are based on a solid carrier and in which a tracer to be transformed into ion form is utilized in a detection reaction, the tracer being separable from the liquid electrically by means of either electrode.

The apparatus according to the invention is characterized in that it comprises a positive and a negative electrode 1 and 2 to be placed in a container 4 and connected to a power supply, and a solid carrier 3 to be positioned in the container, whereupon the positive and negative electrode are positioned in such a way that one electrode is in the centre point of the solid carrier structure to be placed in the container and the other electrode is encircling the carrier. The preferred embodiments of the invention are disclosed in the appended claims 2 to 10.

The apparatus according to the invention is applicable in immunoassays which are based on the use of a solid carrier and in which a tracer is used in the detection, the tracer being separable electrically by means of an electrode. The immunoassay method according to the invention is characterized in that an antibody or antigen is bound to a solid carrier 3 and contacted with a corresponding antigen or antibody which is to be analyzed and which is contained in the sample to be examined, the detection of the corresponding antigen or antibody employing a tracer which is electrically separated by means of an electrode 1 or 2 from electrically conductive liquid. For example if the antibody or antigen is linked with an enzymatic label, changes in the conductivity of a solution as a consequence of an enzymatic reaction can be monitored. In this case, the tracer molecule is a substrate of the particular enzyme or enzymes applied in the detection reaction. The preferred embodiments of the method are disclosed in the appended claims 12 to 19.

According to the invention, a carrier is placed between a positive and a negative electrode in a container with electrically conductive liquid, whereupon an electrically charged tracer is transported to the opposite electrode. Measurement can simply be performed on the electrode instead of performing it from the surface of a loop or some other corresponding immunostrip. The tracer is then enriched and the accuracy of the method is improved. In addition to the possibility of enriching the tracer, the present invention also provides other advantages. When the carrier is preferably convex the formation of aggregates probably decreases, and for example when the carrier has the form of a loop a wash may be performed evenly on the entire surface by rotating the loop in a wash solution. The wash may be controlled and standardized by both the duration of the wash and the rotational velocity of the loop.

### List of figures

In the following, the invention will be described in greater detail by means of one preferred embodiment with reference to the accompanying drawing, in which
Figure 1 is a top view of the apparatus according to the invention,
Figure 2 is a cross-sectional side view of the apparatus according to the invention,
Figures 3a and 3b are cross-sectional side views of the apparatus according to the invention arranged in a microtiter plate,
Figure 4 shows a solid carrier having the form of a loop, and
Figure 5 shows an antibody attached to a loop, its antigen, and an antibody to the antigen labeled with a radioactive metal.

### Detailed description of the invention

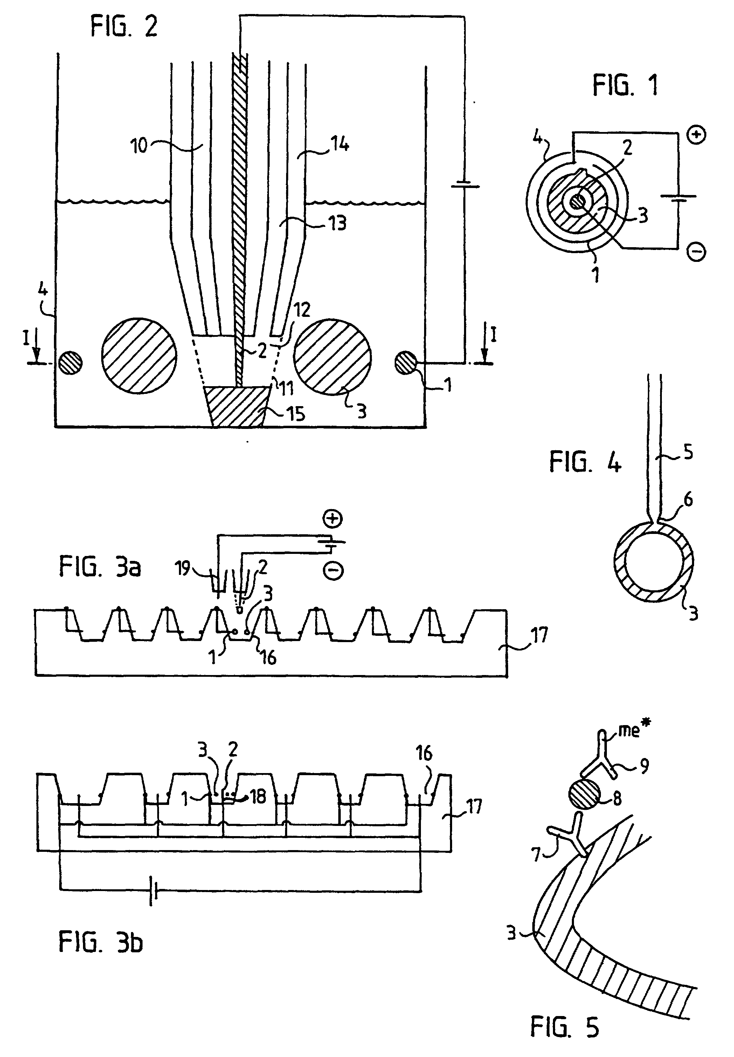

Figures 1 and 2 show the apparatus according to the invention, comprising a positive electrode 1, i.e. an anode, and a negative electrode 2, i.e. a cathode, and a solid carrier 3 placed between them in such a way that it encircles the electrode 2. A power supply is connected to the electrodes. The conventional positive and negative electrodes are used in the sensor.

The other electrode 2 is preferably contained in a tip 14, which also comprises a detection unit 10. The electrode 2 may further be positioned in a space 12 surrounded by a porous wall, for example a semi-permeable membrane 11. A conduit 13 for reagents used in the detection may lead to the space 12. The end of the tip may comprise insulation 15.

The electrode encircling the carrier may be for example annular, as in Figures 1 and 2, or net-like. The apparatus is placed in a container 4 for electrically conductive liquid. The container may preferably be for example an Eppendorff test tube or a well 16 in a microtiter plate 17 shown in Figure 3. One electrode 1 in said microtiter plate is on the wall of the plate. Figure 3a shows a microtiter plate in which one electrode 1 is connected to a conductor 19 as the other electrode 2 is placed in the loop.. This requires a microtiter plate with a special structure comprising separately built-in conductors in each well. It is also possible to install both electrodes 1 and 2 in the well 16 of the microtiter plate 17 or in some other container 4 where the assay is performed. The electrode 2 may then be positioned in a projection 18 protruding upwards from the bottom of the container, the projection being positioned in the middle of the carrier, according to the invention (see Figure 3b).

The solid carrier 3 is constructed in such a way that one electrode may be placed into a solution inside it, into the centre of it, and the other one outside it. The carrier may be for example a perforated ball inside which one of the electrode is placed, but preferably it is in the form of a loop, as shown in Figure 4. The loop preferably comprises an associated handle 5 comprising a cut-off point 6. The cut-off point 6 of the handle 5 comprises a weakened area. The perforated ball may also be realized in such a way that the same carrier comprises several built-in loops positioned spatially at different angles with respect to the ball axis in which the electrode is placed. The circumferences of the circles in these loops positioned together are thereby situated on the same imaginary spherical surface. The carrier 3 may be of any material to which reactant, such as protein, can be bound, for example polyvinyl chloride or polystyrene.

The apparatus according to the invention may be used in different types of immunoassays based on the use of a solid carrier. Both direct and indirect methods of detection are possible, and they may be competitive as well as non-competitive methods. These immunological methods known per se are described at length in the literature of the field. Reference is made here for example to *Principles and Practice of Immunoassay* by C.P. Price & D.J. Newman (Eds.), 1991, Stockton Press, New York, and to *Antibodies - a Laboratory Manual* by E. Harlow & D. Lane, 1988, Cold Spring Harbor Laboratory, USA. The embodiment described below is only one example illustrating the invention, but it is clear for one skilled in the art that the apparatus may also be applied in other types of immunoassay methods.

An antibody 7 to a particular antigen is bound to the carrier 3 (Figure 5). The antibody bound to the carrier is then reacted with an antigen 8 contained in the liquid sample to be tested, whereafter the loop is washed by rotating it in a wash solution. The loop, which comprises an attached antibody and its antigen, is then reacted with an antibody 9 to said antigen, a tracer (Me*), such as a radioactive metal, being connected to this antibody. The tracer has to be transformable into ion form whereby it can be separated electrically from the reaction solution. In the method according to the invention, it is preferable to use as the tracer a metal which due to its positive charge is easily bound to a protein having usually a negative net charge. There are several known manners of connecting metals to proteins.

After the loop has reacted with the antibody 9, it is washed again in order to remove unbound labeled antibody. The loop is then placed in the container 4 comprising electrically conductive reaction solution, and the antibody 9 attached to the loop and labeled with the tracer is subjected to conditions wherein its tracer is transformed into ion form, for example by lowering the pH. The handle of the loop is broken at the cut-off point 6, the electrodes are immersed in the reaction solution and the power is switched on, whereupon the tracer in the electrically charged form is transported to the electrode of opposite charge, in this case to the negative electrode. The electrode to which the tracer is transported is preferably situated inside the loop, and even more preferably it further comprises a connected detection unit 10 capable of indicating the amount of the tracer, which is proportionate to an analyte in the sample to be examined. The tracer may be measured either directly, for example on the basis of its radioactivity, or indirectly. It is also possible to measure it by utilizing the electric phenomena caused by the tracer, for example variation in voltages. In indirect measurement it is possible to use as the tracer for example an agent which in ion form is an activator of an enzyme (for example an enzyme causing a colour reaction) or its inhibitor (e.g. in the aforementioned colour reaction). An example is β-galactosidase, which is inhibited by heavy metals (Johansson, A., 1991. *Heterogenous enzyme immunoassay* In Price, C.P. and Newman, D.J. (Eds.), *Principles and Practice of Immunoassay.* Stockton Press. New York. USA) It is also possible to use for example a Ca-label, whereupon when the antibody complex disassociates, Ca²⁺ aiming at the cathode may act as the activator of a photoprotein activated by calcium. The activated photoprotein emits light, which may be measured optically (Blinks, J.R., 1985. *Detection of Ca*^{2±} *with Photoproteins* In Van Dyke, K. (Ed.), *Bloluminescence and Chemiluminescence: Instruments and Applications,* vol. II. CRC Press Inc. Boca Raton, Florida, USA). When the tracer is for example lanthanide, the lanthanide ion migrated to the cathode may be reacted with certain chelating agents, thus forming very strongly fluorescent chelates (Faix, J.D., 1992. Delfia (*Dissociation-Enhanced Lanthanide Fluoroimmunoassay*) *Analyzer* In Chan, D.W. (Ed.) *Immunoassay Automation: A Practical Guide*. Academic Press. San Diego, USA. p. 151-166; Stȧhlberg, T.; Markela, E.; Mikola, H.; Mottram, P. & Hemmilä, I., 1994. *Europium* and samarium *in time-resolved Fluoroimmunoassays.* International Laboratory Jan/Feb 1994; and Ellis, G., 1992. *CyberFluor Immunoassay System* In Chan, D.W. (Ed.) *Immunoassay Automation: A Practical Guide.* Academic Press. San Diego, USA. p. 137-150).

The detection unit 10 may be an optical fibre or some other sensor or detection means, which measures for example radioactivity, fluorescence, colour, a change of colour in an enzymatic reaction, etc.

It is possible to add, through the conduit 13, reagents required in the detection of the tracer, such as enzymes, Ca²⁺-activated photoproteins, etc. It is also preferable then that the electrode attracting the tracer is included in the space 12 restricted by the semi-permeable membrane 11. Said membrane prevents the reagents from diffusing away from the tracer which is to be detected and which has migrated to the electrode.

It is also possible to connect the electrodes in another manner so that the negative electrode is outside the loop and the positive electrode is inside the loop. The tracer may alternatively be negatively charged, whereupon it migrates to the positive electrode. It is also possible to use more than one tracer, whereupon some of the tracers may migrate to the positive electrode and some to the negative electrode.

## Claims

1. An apparatus for carrier-based immunoassays in which an antibody or antigen is bound to a solid carrier, **characterized in that** it comprises a positive and a negative electrode (1 and 2) to be placed in a container (4) and connected to a power supply, whereupon either the positive or the negative electrode is constructed in such a way that it encircles the other electrode, and a solid carrier (3) to be positioned in between the said electrodes into a liquid in the container.

2. An apparatus according to claim 1, **characterized in that** the carrier (3) to be placed in the container has the form of a loop attached to a breakable handle (5).

3. An apparatus according to claim 1, **characterized in that** that the carrier (3) to be placed in the container has the form of a perforated ball.

4. An apparatus according to any one of claims 1 to 3, **characterized in that** the carrier (3) is of polystyrene.

5. An apparatus according to any one of claims 1 to 4, **characterized in that** one electrode is placed in a tip (14) which comprises a detection unit (10).

6. An apparatus according to any one of claims 1 to 5, **characterized in that** the electrode (2) inside the carrier (3) is arranged to be used for collecting a tracer.

7. An apparatus according to claim 1 or 6, **characterized in that** the electrode (2) is positioned in a space (12) separated by a porous wall (11).

8. An apparatus according to claim 7, **characterized in that** a conduit (13) for reagents used in the detection leads to the space (12).

9. An apparatus according to claim 1, **characterized in that** it comprises a container (4) which is a well (16) in a microtiter plate (17), the electrode (1) being placed in the well in such a way that the electric circuit is switched on simultaneously with the positioning of the other electrode (2) into the liquid inside the solid carrier (3) in the well (16) of the microtiter plate.

10. An apparatus according to claim 1, **characterized in that** it comprises a container (4) which is a well (16) in a microtiter plate (17), one electrode (1) being situated on the wall of the well (16) and the other electrode (2) in a projection (18) protruding upwards from the bottom of the well (16).

11. A method for using the apparatus according to claim 1, **characterized in that** an antibody of antigen is bound to a solid carrier (3) and contacted with a corresponding antigen or antibody which is to be analysed an which is contained in the sample to be tested, the detection of the corresponding antigen or antibody employing a tracer which is transformed into ion form and which is electrically separated by means of two electrodes (1 and 2) from electrically conductive liquid, and the ion migrated to the electrode is assayed as a measure of antibody or antigen to be analysed.

12. A method according to the claim 11, **characterized in that** the detection is based on an enzymatic reaction in which reaction the colour, conductivity, or other measurable character of the liquid is changing, and the change is then measured.

13. A method according to claim 11, **characterized in that** the tracer is a metal which is bound to a protein and which can be transformed into ion form and enriched to the negative electrode where it is measured.

14. A method according to claim 13, **characterized in that** the metal is radioactive.

15. A method according to claim 13, **characterized in that** the metal is optically measured by means of the activation of the said metal of a photoprotein and by means of the light thus emitted.

16. A method according to claim 13, **characterized in that** the metal is measured by means of the activation or inhibition of the said metal of an enzyme protein.

17. A method according to claim 13, **characterized in that** the metal is measured by means of the activation or inhibition of the said metal of a fluorescence phenomenon.

18. A method according to claim 13, **characterized in that** a carrier (3) in the form of a loop is used, a negative electrode (2) being placed in the middle of the carrier.

19. A method according to claim 11, **characterized in that** more than one tracer is used, and some of the tracers may be transported to the positive and some to the negative electrode.

## Patentansprüche

1. Vorrichtung für Immunitätsuntersuchung auf Indikatorbasis, wobei ein Antikörper oder ein Antigen an einen festen Indikator gebunden wird, **dadurch gekennzeichnet, dass** sie eine positive und negative Elektrode (1 und 2) aufweist, die in einen Behälter (4) angeordnet und an eine Stromquelle angeschlossen werden, woraufhin entweder die positive oder negative Elektrode derart konstruiert wird, dass sie die andere Elektrode umfasst und ein fester Indikator (3) zwischen den Elektroden in einer Flüssigkeit im Behälter angeordnet wird.

2. Vorrichtung entsprechend dem Patentanspruch 1, **dadurch gekennzeichnet, dass** der im Behälter angeordnete Indikator (3) die Form einer Schlaufe hat und mit einem abzubrechenden Arm (5) ausgestattet ist.

3. Vorrichtung entsprechend dem Patentanspruch 1, **dadurch gekennzeichnet, dass** der im Behälter angeordnete Indikator (3) die Form einer perforierten Kugel hat.

4. Vorrichtung entsprechend einem der Ansprüche 1 - 3, **dadurch gekennzeichnet, dass** der Indikator aus Polystyrol ist.

5. Vorrichtung entsprechend einem der Ansprüche 1 - 4, **dadurch gekennzeichnet, dass** eine der Elektroden an der Spitze (14) angeordnet ist, die wiederum eine Anzeigeneinheit (10) aufweist.

6. Vorrichtung entsprechend einem der Ansprüche 1 - 5, **dadurch gekennzeichnet, dass** die innerhalb des Indikators (3) vorhandene Elektrode (2) für den Empfang eines Indikators ausgelegt worden ist.

7. Vorrichtung entsprechend dem Patenanspruch 1 oder 6, **dadurch gekennzeichnet, dass** die Elektrode (2) in einem Raum (12) angeordnet ist, der mit einer porösen Wand (11) abgetrennt worden ist.

8. Vorrichtung entsprechend dem Patentanspruch 7, **dadurch gekennzeichnet, dass** in den Raum (12) ein Kanal (13) für die bei der Indikation anzuwendenden Reaktionsmittel geführt wird.

9. Vorrichtung entsprechend dem Patentanspruch 1, **dadurch gekennzeichnet, dass** sie weiter einen Behälter (4) aufweist, der die Mulde (16) der Muldenplatte (17) ist, in der die Elektrode (1) so angeordnet werden kann, dass der Stromkreis gleichzeitig geschaltet wird, wenn die andere Elektrode (2) in der Flüssigkeit innerhalb des festen Indikators (3) in der Mulde der Muldenplatte angeordnet wird.

10. Vorrichtung entsprechend dem Patenanspruch 1, **dadurch gekennzeichnet, dass** sie weiter einen Behälter (4) aufweist, die eine Mulde (16) der Muldenplatte (17) ist, in der die eine Elektrode (1) an der Wand der Mulde (16) und die andere Elektrode (2) an der vom Boden der Mulde (16) herausragenden Konsole (18) angeordnet ist.

11. Verfahren entsprechend dem Patentanspruch 1 für den Betrieb der Vorrichtung, **dadurch gekennzeichnet, dass** an den festen Indikator (3) Gegenmittel oder Antigen gebunden wird, und mit dem entsprechenden Antigen oder Antikörper in Berührung gebracht wird, das analysiert wird und welche in der zu untersuchenden Probe vorhanden ist, die Erkennung des entsprechenden Antigens oder Antikörpers weist einen Indikator auf, welche in Ionenform umgewandelt wird und welcher elektrisch mit Hilfe der zwei Elektroden (1 oder 2) von elektrisch leitenden Flüssigkeit abgeschieden wird und das in die Elektrode geführte Ion als Maß des zu analysierenden Antikörpers oder Antigens definiert wird.

12. Verfahren entsprechend dem Patentanspruch 11, **dadurch gekennzeichnet, dass** die Indikation auf einer Enzymreaktion basiert, in welcher die Farbe, die Leitfähigkeit oder sonstige messbare Eigenschaft der Flüssigkeit geändert wird und die Änderung gemessen wird.

13. Verfahren entsprechend dem Patentanspruch 11, **dadurch gekennzeichnet, dass** als Indikator ein mit Protein verbundenes Metall verwendet wird, das in Ionenform umgewandelt und zu einer negativen Elektrode angereichert werden kann, wo es gemessen wird.

14. Verfahren entsprechend dem Patentanspruch 13, **dadurch gekennzeichnet, dass** das Metall radioaktiv ist.

15. Verfahren entsprechend dem Patentanspruch 13, **dadurch gekennzeichnet, dass** das Metall mit Hilfe der Aktivierung des gesagten Metalls eines Photoproteins und des somit emittierten Lichts optisch gemessen wird.

16. Verfahren entsprechend dem Patentanspruch 13, **dadurch gekennzeichnet, dass** das Metall mit Hilfe der Aktivierung oder Sperrung des gesagten Metalls eines Enzymproteins gemessen wird.

17. Verfahren entsprechend dem Patentanspruch 13, **dadurch gekennzeichnet, dass** das Metall mit Hilfe der Aktivierung oder Sperrung des gesagten Metalls einer Fluoreszenzerscheinung gemessen wird.

18. Verfahren entsprechend dem Patentanspruch 13, **dadurch gekennzeichnet, dass** ein Indikator (3) in Form einer Schlaufe verwendet wird, in dessen Mitte eine negative Elektrode angeordnet ist.

19. Verfahren entsprechend dem Patentanspruch 11, **dadurch gekennzeichnet, dass** mehrere als ein Indikator verwendet werden, von denen ein Teil zu der positiven Elektrode und ein Teil zur negativen Elektrode geführt werden kann.

## Revendications

1. Un équipement pour les essais immunologiques, basés sur le porteur, où un anticorps ou un antigène est lié à un porteur solide, **caractérisé en ce qu'**il comprend une électrode positive et une électrode négative (1 et 2), prévues pour être placées dans un récipient (4) et branchées sur une prise de courant, l'électrode positive ou l'électrode négative étant alors construite de telle manière qu'elle entoure l'autre électrode, et un porteur solide (3) qui est installé entre lesdites électrodes, dans un liquide dans le récipient.

2. Un équipement suivant la revendication 1, **caractérisé en ce que** le porteur (3) à placer dans le récipient a la forme d'une boucle fixée sur un manche cassable (5).

3. Un équipement suivant la revendication 1, **caractérisé en ce que** le porteur (3) à placer dans le récipient a la forme d'une balle perforée.

4. Un équipement suivant l'une des revendications de 1 à 3, **caractérisé en ce que** le porteur (3) est en polystyrène.

5. Un équipement suivant l'une des revendications de 1 à 4, **caractérisé en ce que** l'une des électrodes est placée dans une pointe (14) qui comprend un élément de détection (10).

6. Un équipement suivant l'une des revendications de 1 à 5, **caractérisé en ce que** l'électrode (2) à l'intérieur du porteur (3) est prévue pour être utilisée pour la réception d'un traceur.

7. Un équipement suivant la revendication 1 ou 6, **caractérisé en ce que** l'électrode (2) est installée dans un espace (12) séparé par une paroi poreuse (11).

8. Un équipement suivant la revendication 7, **caractérisé en ce qu'**un conduit (13) pour des réactifs utilisés dans la détection, aboutit à l'espace (12).

9. Un équipement suivant la revendication 1, **caractérisé en ce qu'**il comporte un récipient (4) qui est un puits (16) dans une microplaque (17), l'électrode étant placée dans le puits de telle façon que le circuit électrique est branché simultanément avec l'installation de l'autre électrode (2) dans le liquide à l'intérieur du porteur (3) dans le puits (16) de la microplaque.

10. Un équipement suivant la revendication 1, **caractérisé en ce qu'**il comprend un récipient (4) qui est un puits (16) dans la microplaque (17), une électrode (1) étant placée sur la paroi du puits (16) et l'autre électrode (2) dans une saillie (18) qui avance vers le haut depuis le fond du puits (16).

11. Un procédé pour l'utilisation de l'équipement selon la revendication 1, **caractérisé en ce qu'**un anticorps ou un antigène est lié à un porteur solide (3) et mis en contact avec un antigène ou un anticorps correspondant à analyser, contenu dans l'échantillon à examiner, la détection de l'antigène ou de l'anticorps correspondant étant effectuée à l'aide d'un traceur transformé en forme d'ion, qui est séparé électriquement au moyen de deux électrodes (1 et 2) d'un liquide conducteur d'électricité et l'ion migré à l'électrode est déterminé comme mesure de l'anticorps ou de l'antigène à analyser.

12. Un procédé selon la revendication 11, **caractérisé en ce que** la détection est basée sur une réaction enzymatique dans laquelle la couleur, la conductibilité ou une autre caractéristique mesurable du liquide change, et le changement est ensuite mesuré.

13. Un procédé selon la revendication 11, **caractérisé en ce que** le traceur est un métal qui est lié à une protéine et qui peut être transformé en forme d'ion et enrichi à l'électrode négative où il est mesuré.

14. Un procédé selon la revendication 13, **caractérisé en ce que** le métal est radioactif.

15. Un procédé selon la revendication 13, **caractérisé en ce que** le métal est mesuré optiquement au moyen de l'activation dudit métal d'une photoprotéine et au moyen de la lumière ainsi émise.

16. Un procédé selon la revendication 13, **caractérisé en ce que** le métal est mesuré au moyen de l'activation ou l'inhibition dudit métal d'une protéine enzymatique.

17. Un procédé selon la revendication 13, **caractérisé en ce que** le métal est mesuré au moyen de l'activation ou l'inhibition dudit métal d'un phénomène de fluorescence.

18. Un procédé selon la revendication 13, **caractérisé en ce qu'**un porteur (3) en forme de boucle est utilisé, une électrode négative (2) étant placée au milieu du porteur.

19. Un procédé selon la revendication 11, **caractérisé en ce que** plus qu'un traceur est utilisé et que quelques-uns des traceurs peuvent être transportés à l'électrode positive et d'autres à l'électrode négative.
